# EUROPEAN PATENT SPECIFICATION

(11) **EP 0 684 042 B1**
(45) Date of publication and mention of the grant of the patent: **10.12.1997**
(21) Application number: 95107106.7
(22) Date of filing: 12.05.1995
(51) Int. Cl.: A61K 31/19, A61K 47/20, A61K 9/20

(54) **Oral solid pharmaceutical compositions containing as active ingredient gemfibrozil and process for preparing them**
Feste orale pharmazeutische Zubereitung mit Gemfibrozil als aktiver Inhaltsstoff und Verfahren zur Herstellung
Composition pharmaceutique solide à l'applications orale contenant de gemfibrozil comme ingrédient actif et un procédé pour sa préparation

(30) Priority: 13.05.1994 HU 9401506
(43) Date of publication of application: 29.11.1995
(73) Proprietor: EGIS GYOGYSZERGYAR, H-1106 Budapest (HU)
(72) Inventor: Fekete, Pál, Dr., H-1051 Budapest (HU); Fellner née Köhalmi, Erzsébet, Dr., Budapest XII. (HU); Sándorfalvy, Andrea, H-1156 Budapest (HU); Bezzegh, Dénes, H-1122 Budapest (HU); Ujfalussy, György, Dr., H-1093 Budapest (HU); Gora née Hernyes, Magdolna, H-2117 Isaszeg (HU); Klebovich, Imre, Dr., H-1125 Budapest (HU); Drabant, Sándor, Dr., H-1171 Budapest (HU); Mándi, Attila, Dr., H-1026 Budapest (HU); Maroshelyi née Kovács, Biborka, Dr., H-1165 Budapest (HU); Szánto, Márta, H-1165 Budapest (HU); Szlávy, née Széll, Zsuzsa, Budapest (HU)
(74) Representative: Beszédes, Stephan G., Dr.

(56) References cited:
- EP-A- 0 462 067
- WO-A-93/24154
- US-A- 4 387 107

## Description

The invention is concerned with oral solid pharmaceutical compositions containing as active ingredient gemfibrozil, preferably in the form of tablets, film-coated tablets or capsules, and a process for preparing them.

Gemfibrozil, 5-[2',5'-di-(methyl)-phenoxy]-2,2-di-[methyl]-pentanoic acid, is a widely used antihyperlipoproteinemic agent having a high daily dose ranging between 900 mg and 1 500 mg. This active agent is in water only poorly soluble and has a hydrophobic character. For this reason the preparation of pharmaceutical compositions containing gemfibrozil with adequate dissolution and adsorption of the active ingredient involves serious difficulties.

In the commercially available compositions various surfactants are used to promote the dissolution of the active ingredient. Thus the 300 mg capsule and 600 mg tablet marketed in the USA (manufacturer: Parke-Davis) contains as surfactant polysorbate 80 [mono-9-octadecenoate poly-(oxy-1,2-ethanediyl)-sorbitane derivative] and sodium lauryl sulfate, respectively (Physicians Desk Reference, 45th Edition [1991], publisher: E. R. Barnhart, Oradell, N.Y. USA, page 1 668]. The tablet containing 450 mg gemfibrozil put on the market in Germany under the name "Gevilon®" (by the firm Parke-Davis) also comprises a polysorbate 80 surfactant (Rote Liste 1992, Bundesverband der Pharmazeutischen Industrie, Frankfurt/Main, preparation 57 020).

Immediate and sustained release pharmaceutical compositions containing gemfibrozil have been disclosed in HU-PS 204,192. According to this Prior Art 0.7 to 0.8% by weight of polysorbate 80 (Tween® 80) is used as surfactant in the preparation of granules.

HU-PS 204,194 relates to immediate release gemfibrozil compositions and to compositions from which the active ingredient is delivered in the intestines. Such immediate release granules are prepared by using 0.5% by weight of sodium lauryl sulfate.

HU-PS 204,193 relates to water dispersible compositions containing gemfibrozil. These non-sustained release compositions are prepared by first coating the finely distributed gemfibrozil particles with a mixture of a microcrystalline wax and a hydrophilic agent, e.g. a fatty alcohol, fatty acid ester, polyol, cellulose derivative or vinyl derivative, and thereafter overcoating the particles with 0.5 to 2.0% by weight of a surfactant. For this purpose as surfactant the use of sodium lauryl sulfate is proposed.

According to the evaluation of the Prior Art in published European patent application 462,067 commercially available gemfibrozil capsules contain about 0.2% by weight of sodium lauryl sulfate and commercially available gemfibrozil tablets contain about 0.7% by weight of sodium lauryl sulfate. However, this is not sufficient to ensure a suitable dissolution of the active ingredient. According to the teaching of published European patent application 462,067 immediate release gemfibrozil tablets may be prepared by using a larger amount, namely 1 to 4% by weight, related to the amount of gemfibrozil, of a surfactant having a hydrophilic-lipophilic balance (HLB) between 10 and 50. In the disclosure of said European patent application the following surfactants are enumerated: polysorbates, Pluronic®-type additives (polyoxyethylene-polyoxypropylene copolymers), alkali salts of fatty acid sulfates, particularly sodium lauryl sulfate, salts of fatty acids, e.g. sodium oleate and triethanol amine oleate. In published European patent application 462,067 it is particularly emphasized that such surfactants must be used in an amount of 1 to 4% by weight, related to the gemfibrozil content of the composition.

According to published European patent application 475,894 quick dissolution of gemfibrozil tablets can be attained by using buffer salts of strong bases and weak acids, e.g. carbonates and citrates, as carrier which provide a pH value of above 5 in aqueous medium. However, it appears from said European patent application that such compositions must contain at least 1% by weight of sodium lauryl sulfate, related to the gemfibrozil content, to achieve the desired quick dissolution of the active ingredient.

From US Patent 4,387,107 it has been known a therapeutic aqueous benzoyl peroxide gel composition comprising from about 2.5 to about 15% by weight of micronized benzoyl peroxide having a particle size of less than 150 microns with a mean average particle size in said composition of less than 35 microns and a stabilizing agent which comprises 0.1 to 4% by weight of dioctyl sodium sulfosuccinate.

Furthermore in WO 93/24154 it has been described a biodegradable controlled release composition formed by flash-flow melt-spinning comprising a nonsaccharide biodegradable polymer and a bio-effecting agent. As bio-effecting agent also gemfibrozil has been mentioned. No mention has been made of bis-[2-(ethyl)-hexyl]-sodium-sulfosuccinate.

Surfactants used in orally administered pharmaceutical compositions cannot be regarded to be completely inert auxiliary agents. That is, such additives do not promote only the dissolution and adsorption of the active ingredient but may also increase the dissolution and adsorption in the gastrointestinal tract of certain other, partly toxical substances. For this reason it is desirable to avoid the use of too high amounts of a surfactant. This is particularly true for pharmaceutical compositions containing active ingredients which are to be administered to the patients in large dose and for a longer period of time because thus a larger amount of surfactant and toxical substances may get into the organism.

Taking into consideration that gemfibrozil belongs to the active ingredients which are to be administered in large doses and continuously for a longer period of time, there is a strong demand for pharmaceutical compositions containing gemfibrozil which ensure quick dissolution and adsorption of the active ingredient by using a relatively small amount of a surfactant.

Hence the problem underlying to the invention is to create oral solid pharmaceutical compositions containing as active ingredient gemfibrozil which, while eliminating the disadvantages of the known compositions, contain a relatively low amount of a surfactant, by which the slowing down of the dissolution of the active ingredient taking place particularly on storing at high temperatures is eliminated and which show a small standard deviation in the dissolution velocity of the individual capsules and tablets within a batch and among several batches as well as a process for preparing them.

Surprisingly it has been found that the above problem can be solved in a satisfactory manner by the present invention.

Surprisingly it has been found that a relatively small amount, i.e. 0.05 to 0.5% by weight, related to the gemfibrozil content of the composition, of bis-[2-(ethyl)-hexyl]-sodium-sulfosuccinate (also designed as "Diotilan") ensures a very quick and uniform dissolution of the active ingredient gemfibrozil from tablets or capsules. The standard deviation of dissolution within a given batch and among different batches is very small. Furthermore the slowing down of the dissolution velocity taking place particularly on longer storing at high temperatures is efficiently prevented by the addition of bis-[2-(ethyl)-hexyl]-sodium-sulfosuccinate in the above disclosed amounts.

Hence a subject matter of the invention are oral solid pharmaceutical compositions containing as active ingredient gemfibrozil and 1 or more conventional pharmaceutical auxiliary agent(s) and a surfactant, which are characterized in that they contain as surfactant bis-[2-(ethyl)-hexyl]-sodium-sulfosuccinate in an amount of from 0.05 to 0.5% by weight, related to the gemfibrozil content of the composition.

The above advantageous effects of bis-[2-(ethyl)-hexyl]-sodium-sulfosuccinate used in such a relatively low concentration are unexpected and could not be foreseen in the light of the Prior Art. According to published European patent application 462,067 a suitable chick gemfibrozil dissolution can be achieved only by using at least 1% by weight of a hydrophilic surfactant having a hydrophilic-lipophilic balance (HLB) between 10 and 50. Bis-[2-(ethyl)-hexyl]-sodium-sulfosuccinate also belongs to the grow of surfactants of hydrophilic character. However, published European patent application 462,067 is completely silent in making any reference to the use of bis-[2-(ethyl)-hexyl]-sodium-sulfosuccinate. Hence this European patent application is a prejudice against the present invention.

Preferably the amount of bis-[2-(ethyl)-hexyl]-sodium-sulfosuccinate is from 0.1 to 0.4% by weight of the gemfibrozil content of the composition.

In the gemfibrozil compositions according to the present invention as [an] auxiliary agent(s) such generally used in the manufacture of tablets and capsules are contained.

Thus preferably they contain as [an] auxiliary agent(s) [a] filler(s), particularly microcrystalline cellulose, lactose, mannitol, starch, cellulose and/or calcium phosphate.

It is also preferred that they contain as [an] auxiliary agent(s) [a] binding agent(s), particularly gelatine, polyvinyl pyrrolidone, hydroxypropylmethyl cellulose, polyvinyl alcohol and/or polyvinyl butyral.

Furthermore it is preferred that they contain as [an] auxiliary agent(s) [a] disintegrating agent(s), particularly starch, [an] alkali carboxymethyl starch(es), particularly sodium carboxymethyl starch, [an] alkali carboxymethyl cellulose(s), particularly sodium carboxymethyl cellulose, and/or cross-linked polyvinyl pyrrolidone.

Moreover it is preferred that they contain as [an] auxiliary agent(s) [a] lubricant(s), particularly magnesium stearate, calcium stearate, stearic acid, hydrogenated castor oil and/or talc.

It is also preferred that they contain as [an] auxiliary agent(s) [a] sliding agent(s), particularly colloidal silicic acid and/or talc.

Advantageously the lubricant(s) and/or sliding agent(s) and/or a part of the disintegrating agent(s) are in an external layer.

Preferably the pharmaceutical compositions of the present invention are in the form of tablets, capsules or film-coated tablets.

A further subject matter of the invention is a process for preparing the pharmaceutical compositions according to the invention comprising mixing gemfibrozil as active ingredient and 1 or more conventional pharmaceutical auxiliary agent(s) and a surfactant, which is characterized by using as surfactant bis-[2-(ethyl)-hexyl]-sodium-sulfosuccinate in an amount of 0.05 to 0.5%, particularly 0.1 to 0.4%, by weight, related to the gemfibrozil content of the composition.

In order to prepare a capsule, tablet or film-coated tablet containing gemfibrozil according to the present invention advantageously it is proceeded as follows. The active ingredient is homogenized in dry form with up to 40% by weight of [a] filling agent(s), e.g. cellulose, lactose, mannitol, starch, microcrystalline cellulose and/or calcium phosphate, related to the capsule filling or the uncoated tablet core. If desired, the dry homogenisate may be granulated in a manner known per se. Granulation may be carried out both by the dry and wet procedures. In case of the "dry procedure" the homogenisate is admixed with not more than 6% by weight, preferably not more than 5% by weight, of [a] capsulating or tabletting binding agent(s), e.g. polyvinyl pyrrolidone, hydroxypropyl cellulose, polyvinyl butyral, hydroxypropylmethyl cellulose and/or gelatine, and from 0.05 to 0.5% by weight of bis-[2-(ethyl)-hexyl]-sodium-sulfosuccinate, related to the amount of gemfibrozil, the mixture is converted into briquettes by pressing or transformed into tablets, ground and sieved to the desired particle size, e.g. 0.1 to 1.0 mm. "Wet granulation" may be performed by kneeding the homogenisate with a solution of not more than 6% by weight, preferably not more than 5% by weight, of [a] capsulating or tabletting binding agent(s), e.g. polyvinyl pyrrolidone, hydroxypropylmethyl cellulose, gelatine and/or polyvinyl alcohol, and 0.05 to 0.5% by weight of bis-[2-(ethyl)-hexyl]-sodium-sulfosuccinate, related to the amount of gemfibrozil, formed with water or a C₁₋₃ alkanol or a mixture of water and a C₁₋₃ alkanol. One may also proceed by spraying the solution of the binding agent and bis-[2-(ethyl)-hexyl]-sodium-sulfosuccinate onto the powder mixture of the homogenisate fluidized in a fluidization-type granulating apparatus. The granules thus obtained are dried and sieved to the desired particle size, generally below 1.0 mm. The homogenisate or the granules prepared therefrom are then admixed with [a] lubricant(s), e.g. magnesium stearate, calcium stearate, stearic acid, hydrogenated castor oil and/or talc and/or [a] sliding agent(s), e.g. colloidal silicic acid and/or talc, capsuled or tabletted, whereby, if desired, the tablets may be film-coated by using [a] water soluble polymer(s), e.g. hydroxypropylmethyl cellulose and/or polyethylene glycol, and optionally 1 or more pigment(s), e.g. titanium dioxide, and/or further active ingredient(s), e.g. poly-(dimethyl)-siloxane activated with silicium dioxide {simethicone}.

The quality of the composition may be characterized by the uniformity of the dissolution of the active ingredient and the uniformity of dissolution velocity. The gemfibrozil dissolution of the compositions is determined with the aid of the "paddle" method, disclosed in USP XXII, at 37°C using 900 ml of a phosphate buffer (pH 7.4) as dissolving medium. The amount of the released gemfibrozil is determined by means of high pressure liquid chromatography [HPLC].

The amount of gemfibrozil released from the compositions is determined immediately, at the beginning of the dissolution test, and after 5, 15, 30 and 45 minutes, respectively. Six repetitions were used for each batch (capsules, film-coated tablets, tablets) and the average percentage amount of gemfibrozil dissolved from each tablet or the average percentage amount of gemfibrozil dissolved from six compositions is graphically plotted.

The uniformity of dissolution is characterized by calculating at a given point of time the relative standard deviation (rsd) of the percentage amount of dissolved gemfibrozil within one single batch, the average value of the dissolution from 6 batches each and the standard deviation of the average values among the different batches (RSD). These values are shown in diagrams and compared with the dissolution data, rsd and RSD values of solid pharmaceutical compositions containing commercially available gemfibrozil.

The rsd and RSD values are calculated on the basis of the following equations:$\begin{matrix}\begin{matrix}\text{Average (X) =} \frac{\text{Σ} \text{x}}{\text{n}} \\ \text{Deviation (s) =} \frac{\text{n} \text{·Σ} {\text{x}}^{\text{2}} \text{- (Σ} \text{x} {\text{)}}^{\text{2}}}{\text{n}} \\ \text{rsd =} \frac{\text{s}}{\text{x}} \text{· 100} \\ \text{Deviation of average values (S) =} \frac{\text{N} \text{·Σ} {\text{x}}^{\text{2}} \text{- (Σ} \text{x} {\text{)}}^{\text{2}}}{\text{N}} \\ \text{RSD =} \frac{\text{S} \text{·} \text{N}}{\text{X}} \text{· 100 ,}\end{matrix}\end{matrix}$ wherein
- x =: measured dissolution values (% by weight),
- n =: number of parallel measurements (repetitions, 6 for each batch),
- X =: average dissolution value (per batch),
- N =: number of tested batches,
- s =: standard deviation of dissolution values (within one batch),
- S =: standard deviation of dissolution values (among the different batches),
- rsd =: percentage standard deviation (within one batch) and
- RSD =: percentage standard deviation (among the different batches).

The invention is further illustrated by the following Examples.

### Example 1

### Gemfibrozil capsules

Gemfibrozil capsules having the following composition were prepared:

| Component | Amount mg/capsule |
|---|---|
| Gemfibrozil | 300 |
| Maize starch | 63 |
| Hydroxypropylmethyl cellulose | 16 |
| Magnesium stearate | 6 |
| Sodium carboxymethyl starch | 12 |
| Colloidal silicic acid | 2.4 |
| Bis-[2-(ethyl)-hexyl]-sodium-sulfosuccinate {Diotilan} | 0.4 |

The gemfibrozil and maize starch were homogenized in a Lödige mixer and thereafter granulated with the aqueous solution of bis-[2-(ethyl)-hexyl]-sodium-sulfosuccinate {Diotilan} and hydroxypropylmethyl cellulose. The granules were dried, screened over a 0.8 mm sieve screen and returned into the Lödige apparatus, whereupon the components of the external layer (magnesium stearate, sodium carboxymethyl starch and colloidal silicic acid) were added and the mixture was homogenized. The homogenized mixture was then filled into hard gelatine capsules, size "0".

The release of the active ingredient (dissolution) was determined according to the "paddle" method disclosed in USP XXII, at 37°C, by using 900 ml of a phosphate buffer (pH 7.4) as dissolving medium. The amount of the released gemfibrozil was determined by high pressure liquid chromatography [HPLC].

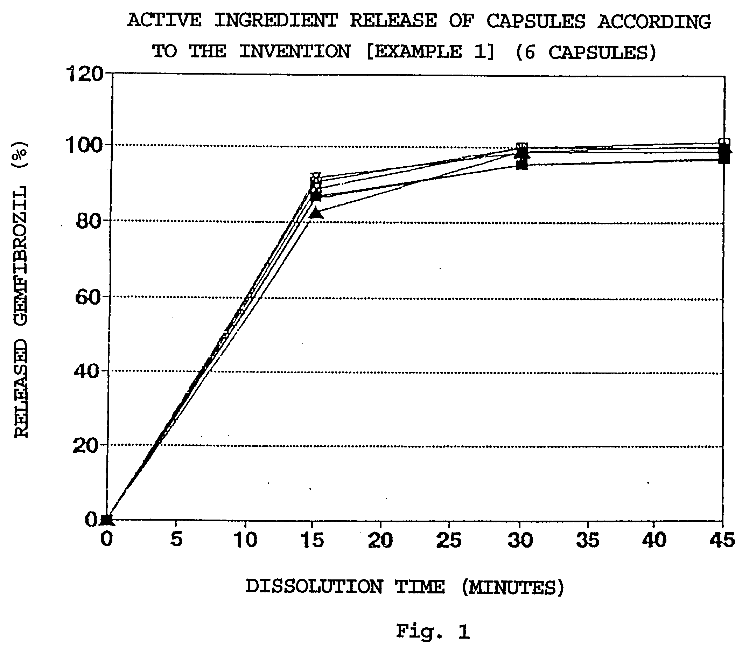

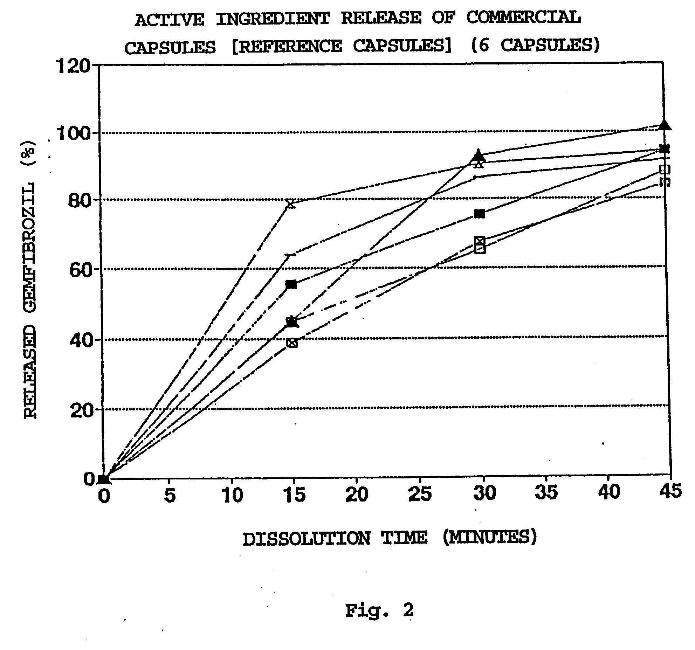

The dissolution data of the capsules prepared according to the invention are disclosed and compared with those of commercially available (US) capsules in Fig. 1 (capsules according to the present invention) and Fig. 2 (commercially available capsules [reference capsules]).

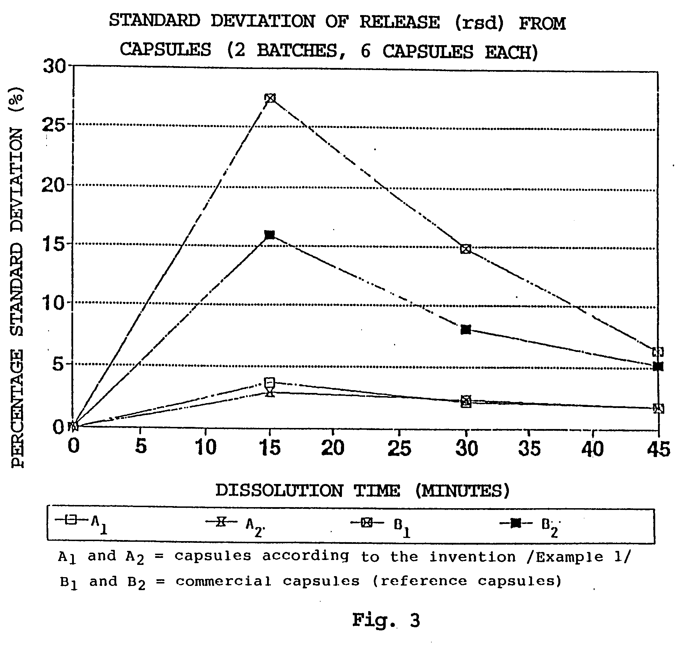

The standard deviation of each batch (6 capsules were used for the dissolution test) was calculated from the experimental data. The time dependence of the rsd values is shown in Fig. 3.

It appears from the above data that the active ingredient dissolution within one batch is more uniform (smaller standard deviation) in case of the capsules according to the invention (A₁ and A₂) than in case of the commercially available reference capsules (B₁ and B₂).

### Example 2

### Gemfibrozil film-coated tablets

Gemfibrozil containing film-coated tablets having the following composition were prepared:

| Component | Amount mg/tablet |
|---|---|
| Gemfibrozil | 600.0 |
| Microcrystalline cellulose | 120.0 |
| Gelatine | 40.0 |
| Bis-[2-(ethyl)-hexyl]-sodium-sulfosuccinate {Diotilan} | 2.0 |
| Calcium stearate | 16.0 |
| Sodium carboxymethyl starch | 54.0 |
| Talc | 24.0 |
| Colloidal silicic acid | 8.0 |

### Film-coating

| Component | Amount mg/tablet |
|---|---|
| Hydroxypropylmethyl cellulose | 9.5 |
| Polyethyleneglycol | 4.0 |
| Poly-(dimethyl)-siloxane activated with silicium dioxide {Simethicone} | 0.5 |
| Titanium dioxide | 2.0 |
| Total weight: | $\overline{\text{880.0 mg}}$ |

The gemfibrozil and microcrystalline cellulose were homogenized in a Lödige whirlpool mixer and the homogenisate was granulated with a mixture of an aqueous solution of gelatine (pH 3 to 4) and an ethanolic solution of bis-[2-(ethyl)-hexyl]-sodium-sulfosuccinate {Diotilan}. The granules were dried, screened through a 0.8 mm sieve screen, returned into the Lödige mixer and homogenized with the components of the external layer (calcium stearate, sodium carboxymethyl starch, talc and colloidal silicic acid). The homogenized mixture was pressed into oval biconvex tablets weighing 864 mg. The tablets are coated in a dragée vessel with a water soluble film by spraying onto the tablets an aqueous dispersion of the above coating components.

The dissolution of the active ingredient was determined by the method described in USP XXII by the "paddle" method, at 37°C, by using 900 ml of a phosphate buffer (pH 7.4) as dissolving medium. The amount of the released gemfibrozil was measured by high pressure liquid chromatography [HPLC].

The dissolution data of the film-coated tablets according to the invention and those of commercially available (US) film-coated tablets were determined immediately after manufacture and also after storing at 40°C for 3 months. For both the film-coated tablets according to the invention and manufactured film-coated tablets 3 batches were used each for the test and each batch consisted of 6 film-coated tablets.

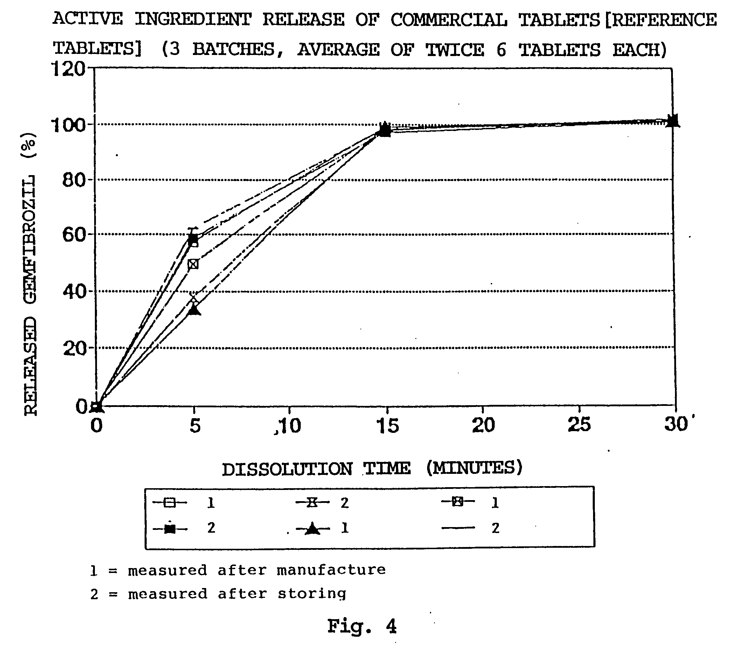

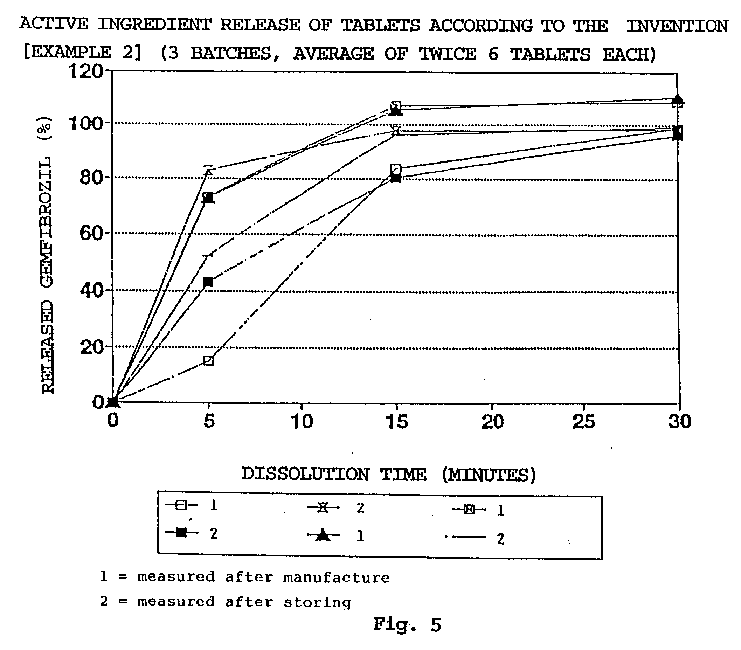

In Fig. 4 in case of the tablets according to the invention data obtained for 3 batches consisting of 6 tablets each are presented. Average data obtained immediately after manufacture and after storing at 40°C for 3 months, respectively, are shown. Fig. 5 displays the corresponding data obtained for 3 batches of the commercially available tablets [reference tablets].

The diagrams clearly show that the dissolution velocity from the commercially available film-coated tablets significantly decreases after storing. Moreover, in commercially available samples the average values of dissolution measured in various batches show a much higher standard deviation than those obtained for the tablets according to the invention.

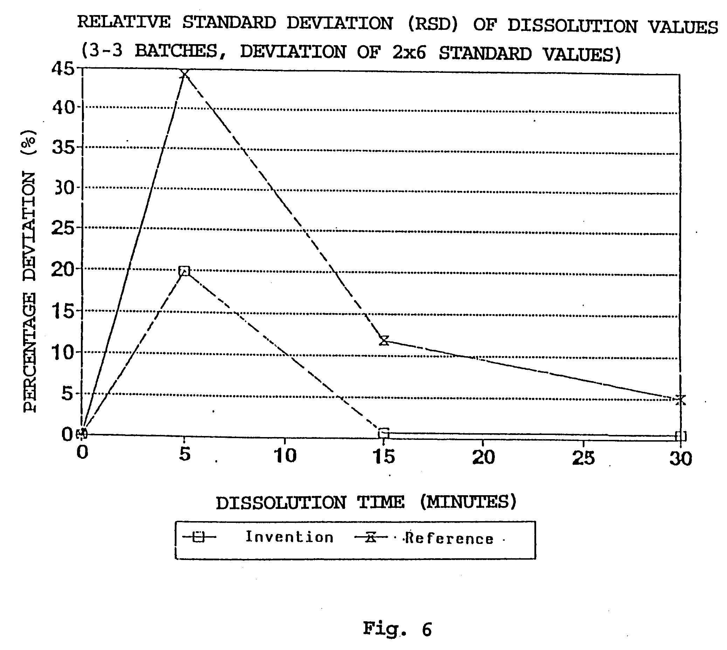

In order to characterize the uniform dissolution more precisely the relative standard deviation (RSD) of the average values of the individual batches of the film-coated tablets according to the invention and of commercially available film-coated tablets [reference tablets] were calculated and the values obtained are plotted against the time. The results are shown in Fig. 6.

It can be seen from Fig. 6 that the standard deviation of the dissolution among the individual batches is significantly lower in case of the product according to the invention than in case of the commercially available reference product.

### Example 3

### Comparative test

The dissolution velocity of tablets containing bis-[2-(ethyl)-hexyl]-sodium-sulfosuccinate {Diotilan} according to the invention, tablets containing an identical amount of sodium lauryl sulfate and tablets containing no surfactant was determined and compared.

Tablets were prepared according to Example 2, except that no film-coating was applied (Experiment 3A). According to Experiment 3B [reference tablet I] bis-[2-(ethyl)-hexyl]-sodium-sulfosuccinate {Diotilan} was replaced by the same amount of sodium lauryl sulfate. According to Experiment 3C [reference tablet II] no surfactant was added to the granulating solution. In Experiments 3A and 3B the weight of the tablets amounted to 864 mg and in Experiment 3C to 862 mg.

The dissolution of the tablets was measured as described in Example 2.

The dissolution results are summarized in the following Table.

**Table**

| Time of dissolution | Experiment 3A 0.33% by weight of bis-[2-(ethyl)-hexyl]-sodium-sulfosuccinate {Diotilan}, related to the gemfibrozil content of the composition [according to Example 2 of the invention] | Experiment 3B 0.33% by weight of sodium lauryl sulfate, related to the gemfibrozil content of the composition [reference tablet I] | Experiment 3C No surfactant [reference tablet II] |
|---|---|---|---|
| 5 minutes | 80% by weight | 36% by weight | 14% by weight |
| 15 minutes | 100% by weight | 81% by weight | 39% by weight |
| 30 minutes | 100% by weight | 100% by weight | 64% by weight |
| 45 minutes | 100% by weight | 100% by weight | 81% by weight |
| 60 minutes | | | 90% by weight |
| 75 minutes | | | 100% by weight |

The above data clearly demonstrate that in the absence of a surfactant the dissolution velocity is very low. A 0.33% by weight amount of sodium lauryl sulfate results in an increase of dissolution velocity but the results obtained with bis-[2-(ethyl)-hexyl]-sodium-sulfosuccinate {Diotilan} in the tablets according to the invention give much better results. It is surprising that bis-[2-(ethyl)-hexyl]-sodium-sulfosuccinate {Diotilan} in the low concentration used provides such a quick release and dissolution of the active ingredient gemfibrozil.

## Claims

1. Oral solid pharmaceutical compositions containing as active ingredient gemfibrozil and 1 or more conventional pharmaceutical auxiliary agent(s) and a surfactant, characterized in that they contain as surfactant bis-[2-(ethyl)-hexyl]-sodium-sulfosuccinate in an amount of from 0.05 to 0.5% by weight, related to the gemfibrozil content of the composition.

2. Pharmaceutical compositions according to claim 1, characterized in that the amount of bis-[2-(ethyl)-hexyl]-sodium-sulfosuccinate is from 0.1 to 0.4% by weight of the gemfibrozil content of the composition.

3. Pharmaceutical compositions according to claim 1 or 2, characterized in that they contain as [an] auxiliary agent(s) [a] filler(s), particularly microcrystalline cellulose, lactose, mannitol, starch, cellulose and/or calcium phosphate.

4. Pharmaceutical compositions according to claims 1 to 3, characterized in that they contain as [an] auxiliary agent(s) [a] binding agent(s), particularly gelatine, polyvinyl pyrrolidone, hydroxypropylmethyl cellulose, polyvinyl alcohol and/or polyvinyl butyral.

5. Pharmaceutical compositions according to claims 1 to 4, characterized in that they contain as [an] auxiliary agent(s) [a] disintegrating agent(s), particularly starch, [an] alkali carboxymethyl starch(es), [an] alkali carboxymethyl cellulose(s) and/or cross-linked polyvinyl pyrrolidone.

6. Pharmaceutical compositions according to claims 1 to 5, characterized in that they contain as [an] auxiliary agent(s) [a] lubricant(s), particularly magnesium stearate, calcium stearate, stearic acid, hydrogenated castor oil and/or talc.

7. Pharmaceutical compositions according to claims 1 to 6, characterized in that they contain as [an] auxiliary agent(s) [a] sliding agent(s), particularly colloidal silicic acid and/or talc.

8. Pharmaceutical compositions according to claims 1 to 7, characterized in that they are in the form of capsules, tablets or film-coated tablets.

9. Process for preparing the pharmaceutical compositions according to claims 1 to 8, comprising mixing gemfibrozil as active ingredient and 1 or more conventional pharmaceutical auxiliary agent(s) and a surfactant, characterized by using as surfactant bis-[2-(ethyl)-hexyl]-sodium-sulfosuccinate in an amount of 0.05 to 0.5% by weight, related to the gemfibrozil content of the composition.

10. Process according to claim 9, characterized by using the bis-[2-(ethyl)-hexyl]-sodium-sulfosuccinate in an amount of 0.1 to 0.4% by weight, related to the gemfibrozil content of the composition.

## Patentansprüche

1. Orale feste pharmazeutische Zusammensetzungen, enthaltend als aktiven Bestandteil Gemfibrozil und 1 oder mehrere herkömmliche pharmazeutische Hilfsstoffe und ein oberflächenaktives Mittel, dadurch gekennzeichnet, daß sie als oberflächenaktives Mittel Bis-[2-(ethyl)-hexyl]-natrium-sulfosuccinat in einer Menge von 0,05 bis 0,5 Gew.-%, in bezug auf den Gemfibrozil-Gehalt der Zusammensetzung, enthalten.

2. Pharmazeutische Zusammensetzungen gemäß Anspruch 1, dadurch gekennzeichnet, daß die Menge an Bis-[2-(ethyl)-hexyl]-natrium-sulfosuccinat 0,1 bis 0,4 Gew.-%, bezogen auf den Gemfibrozil-Gehalt der Zusammensetzung, beträgt.

3. Pharmazeutische Zusammensetzungen gemäß Anspruch 1 oder 2, dadurch gekennzeichnet, daß sie als [ein] Hilfsstoff(e) [ein] Füllstoff(e), insbesondere mikrokristalline Cellulose, Lactose, Mannitol, Stärke, Cellulose und/oder Calciumphosphat, enthalten.

4. Pharmazeutische Zusammensetzungen gemäß Anspruch 1 bis 3, dadurch gekennzeichnet, daß sie als [ein] Hilfsstoff(e) [ein] Bindemittel, insbesondere Gelatine, Polyvinylpyrrolidon, Hydroxypropylmethylcellulose, Polyvinylalkohol und/oder Polyvinylbutyral, enthalten.

5. Pharmazeutische Zusammensetzungen gemäß Anspruch 1 bis 4, dadurch gekennzeichnet, daß sie als [ein] Hilfsstoff(e) [ein] den Zerfall bewirkendes Mittel, insbesondere Stärke, [eine] Alkalicarboxymethylstärke(n), [eine] Alkalicarboxymethylcellulose(n) und/oder vernetztes Polyvinylpyrrolidon, enthalten.

6. Pharmazeutische Zusammensetzungen gemäß Anspruch 1 bis 5, dadurch gekennzeichnet, daß sie als [ein] Hilfsstoff(e) [ein] Lubrikanz(ien), insbesondere Magnesiumstearat, Calciumstearat, Stearinsäure, hydriertes Castoröl und/oder Talkum, enthalten.

7. Pharmazeutische Zusammensetzungen gemäß Anspruch 1 bis 6, dadurch gekennzeichnet, daß sie als [ein] Hilfsstoff(e) [ein] Gleitmittel, insbesondere kolloidale Kieselsäure und/oder Talkum, enthalten.

8. Pharmazeutische Zusammensetzungen gemäß Anspruch 1 bis 7, dadurch gekennzeichnet, daß sie in der Form von Kapseln, Tabletten oder filmbeschichteter Tabletten vorliegen.

9. Verfahren zur Herstellung der pharmazeutischen Zusammensetzungen gemäß Anspruch 1 bis 8, umfassend das Mischen von Gemfibrozil als aktiven Bestandteil und 1 oder mehreren herkömmlichen pharmazeutischen Hilfsstoffen und einem oberflächenaktiven Mittel, gekennzeichnet durch die Verwendung von Bis-[2-(ethyl)-hexyl]-natrium-sulfosuccinat als obenflächenaktives Mittel in einer Menge von 0,05 bis 0,5 Gew.-%, in bezug auf den Gemfibrozil-Gehalt der Zusammensetzung.

10. Verfahren gemäß Anspruch 9, gekennzeichnet durch die Verwendung von Bis-[2-(ethyl)-hexyl]-natrium-sulfosuccinat in einer Menge von 0,1 bis 0,4 Gew.-%, in bezug auf den Gemfibrozil-Gehalt der Zusammensetzung.

## Revendications

1. Compositions pharmaceutiques solides destinées à l'administration par voie orale contenant en tant que principe actif du gemfibrozil et un ou plusieurs agents auxiliaires pharmaceutiques classiques ainsi qu'un agent tensioactif, caractérisées en ce qu'elles contiennent en tant qu'agent tensioactif du bis-[2-(éthyl)-hexyl]-sulfosuccinate de sodium en une quantité de 0,05 à 0,5% en poids par rapport à la teneur de gemfibrozil de la composition.

2. Compositions pharmaceutiques selon la revendication 1, caractérisées en ce que la quantité de bis-[2-(éthyl)-hexyl]-sulfosuccinate de sodium représente de 0,1 à 0,4% en poids par rapport à la teneur de gemfribrozil de la composition.

3. Compositions pharmaceutiques selon la revendication 1 ou 2. caractérisées en ce qu'elles contiennent, en tant qu'agent auxiliaire, une ou plusieurs charges, en particulier: cellulose microcristalline, lactose, manitol, amidon, cellulose, et/ou phosphate de calcium.

4. Compositions pharmaceutiques selon les revendications 1 à 3, caractérisées en ce qu'elles contiennent, en tant qu'agent auxiliaire, un ou plusieurs agents liants, en particulier: gélatine, polyvinylpirrolidone, hydroxypropylméthylcellulose, polyvinylalcool, et/ou polyvinylbutyral.

5. Compositions pharmaceutiques selon les revendications 1 à 4, caractérisées en ce qu'elles contiennent en tant qu'agent auxiliaire, un ou plusieurs agents de désintégration, en particulier: amidon, carboxyméthylamidon(s) d'alcalin(s), carboxyméthylcellulose(s) d'alcalin(s) et/ou polivynyl pyrrolidone réticulée.

6. Compositions pharmaceutiques selon les revendications 1 à 5, caractérisées en ce qu'elles contiennent, en tant qu'agent auxiliaire, un ou plusieurs lubrifiants, en particulier: stéarate de magnésium, stéarate de calcium, acide stéarique, huile de ricin hydrogénée et/ou talc.

7. Compositions pharmaceutiques selon l'une des revendications 1 à 6. caractérisées en ce qu'elles contiennent, en tant qu'agent auxiliaire, un ou plusieurs agents de glissement, en particulier acide silicique colloïdal et/ou talc.

8. Compositions pharmaceutiques selon les revendications 1 à 7, caractérisées en ce qu'elles se présentent sous la forme de gélules, comprimés, ou comprimés enrobés d'un film.

9. Procédé de préparation de compositions pharmaceutiques selon les revendications 1 à 8, comprenant le mélange du gemfribrozil, en tant que principe actif, et d'un ou plusieurs agents auxiliaire pharmaceutiques classiques d'un tensioactif caractérisé en ce qu'on utilise, en tant qu'agent tensioactif du bis-[2-(éthyl)-hexyl]-sulfosuccinate de sodium en une quantité de 0,05 à 0,5% en poids, par rapport à la teneur de gemfribrozil de la composition.

10. Procédé selon la revendication 9, caractérisé en ce que l'on utilise le bis-[2-(éthyl)-hexyl]-sulfosuccinate de sodium en une quantité de 0,1 à 0,4% en poids par rapport à la teneur de gemfibrozil de la composition.
